# EUROPEAN PATENT APPLICATION

(11) **EP 0 941 726 A1**
(43) Date of publication of application: **15.09.1999**
(21) Application number: 99104514.7
(22) Date of filing: 06.03.1999
(51) Int. Cl.: A61F 13/02

(54) **A dressing**

(30) Priority: 12.03.1998 DK 34198
(71) Applicant: COLOPLAST A/S, 3050 Humlebaek (DK)
(72) Inventor: Nielsen, John Stern, 3450 Alleroed (DK)
(74) Representative: Nilausen, Kim (DK)

(57) **Abstract**

A dressing comprising a proximal wound contacting surface covered wholly or partly by a skin-friendly adhesive, a distal surface comprising a non-adhesive backing film, said backing film at least partly being covered by a cover layer wherein said cover layer comprises at least one incision line, and wherein said incision line stretches over the surface of the dressing leaving an uninterrupted zone at the edge of the dressing, is simple and convenient to adapt and apply to the sacrum.

## Description

### FIELD OF THE INVENTION

The present invention relates to a medical dressing, a method of applying such a dressing and a method of preparing such a dressing.

### BACKGROUND OF THE INVENTION

Conventional dressings are usually planar and rectangular or square and may be cut into various shapes to enable them to be more easily applied to a body surface.

However, it is nevertheless sometimes difficult for such dressings to be applied to a body surface, especially a surface in a retracted area, in a manner which avoids creasing and wrinkling of the skin or the dressing or both. This is particular the case where the dressing is provided with an adhesive coating over some or all of its body facing surface. Furthermore, even if creasing or wrinkling can be avoided, if the body surface to which the dressing is to be applied is contoured then it can be difficult and time consuming to apply the dressing in an accurate manner over the contours.

Especially when treating sacral wounds it is often the case that the patient is in a lateral position, which often makes it necessary for the nurse to use one hand to lift natis or stretch the skin of the patient and at the same time use the other hand to apply the dressing. The commercially available products on the market today, are seldom or never adapted to one-hand application. Using only one hand to handle a conventional dressing will often result in unintentional touching of the adhesive part, causing contamination and reduced tackiness, as well as wrinkling and creasing or even the risk of dropping the dressing.

In European Patent application No. 768071 is disclosed a dressing especially for sacral wounds in which the dressing is made more flexible for application to contoured parts of the body by making indented lines or grooves in the dressing, particularly along the central axis of the dressing. These indentations makes it easier to bend the dressing in the desired conformation in order to contacting the wound. This invention still does not solve the problem about handling the dressing with one hand, it will still be difficult to support and control the dressing with only one hand.

In DK patent Specification No. 159370 is disclosed a dressing with a cover layer on top of a backing film. This cover layer is extending beyond the edge of the dressing on two sides and provides in that way two grips for handling. The adhesive side of the backing film is covered with a release liner, divided in two by a incision line extending across the dressing, from one edge to the other. The incision line is present in a wave-form, which when the dressing is bended, will provide several tabs or grips (the top of the waves) easing the release of the release liner without damaging the adhesive.
The process of adding a cover film that is larger than the dressing is technically complicated, and an easy handling of the dressing with one hand will still be difficult.

International patent application No. WO 95/14451 discloses a dressing for application with one hand. On the upper side of this dressing is attached a loop in which the hand of the user can be received. This loop is preferably attached to the dressing by the use of repositionable tape or by a hook and eye system, such as VELCRO™ material. After the application of the dressing, the loop can either be left on the dressing or be detached again. Neither of these solutions are optimal since leaving the loop on the dressing will optionally cause buckles and be uncomfortable for the patient and enhance the friction of the dressing, while tearing off the loop may rip off the dressing or part of it, and can cause nuisance or even damage to the patient. Furthermore, attaching the strap during the production of the dressing, or right before use, is an extra cost incurring step in the process. The strap is a separate part of the dressing, not an integrated part.

It has been found that it is possible to overcome or alleviate some of the above mentioned disadvantages using a dressing of the invention comprising a proximal wound contacting surface covered wholly or partly by a skin-friendly adhesive, a distal surface comprising a non-adhesive backing film, said backing film at least partly being covered by a cover layer wherein said cover layer comprises at least one incision line, and wherein said incision line stretches over the surface of the dressing leaving an uninterrupted zone at the edge of the dressing.

### BRIEF DESCRIPTION OF THE INVENTION

The invention relates to a dressing comprising a proximal wound contacting surface covered wholly or partly by a skin-friendly adhesive, a distal surface comprising a non-adhesive backing film, said backing film at least partly being covered by a cover layer wherein said cover layer comprises at least one incision line.

The invention also relates to a method of application of a dressing comprising a proximal wound contacting surface covered wholly or partly by a skin-friendly adhesive, a distal surface comprising a non-adhesive backing film, said backing film at least partly being covered by a cover layer wherein said cover layer comprises at least one incision line.

The invention also relates to a method of preparing a dressing comprising a proximal wound contacting surface covered wholly or partly by a skin-friendly adhesive, a distal surface comprising a non-adhesive backing film, said backing film at least partly being covered by a cover layer wherein said cover layer comprises at least one incision line.

### BRIEF DESCRIPTION OF THE DRAWINGS

The invention is explained more in detail with reference to the drawings in which:
Figure 1 shows a cross-section of an embodiment of the invention.
Figure 2 shows a top view of an embodiment of the invention.
Figure 3-5 shows the use of an embodiment of the invention.
Figure 6-10 shows different embodiments of the invention.

### DETAILED DESCRIPTION OF THE INVENTION

The invention relates to a dressing comprising a proximal wound contacting surface covered wholly or partly by a skin-friendly adhesive, a distal surface comprising a non-adhesive backing film, said backing film at least partly being covered by a cover layer wherein said cover layer comprises at least one incision line, and wherein said incision line stretches over the surface of the dressing leaving an uninterrupted zone at the edge of the dressing.

The incision line(s) enables the formation of a pocket or strap. Preferably two incision lines are present in the cover layer enabling the formation of a strap.

The incision lines are suitably linear or curved which renders it more easy to form a pocket or strap.

The incision lines are preferably substantially parallel, enabling a simple formation of a strap.

The incision lines are placed in such a manner that when the dressing is bended to a concave conformation having the wound-contacting surface outwards, the part of the cover layer next to the incision lines will rise to form an pocket or strap, in which a body part such as a hand or one or more fingers can be received and thereby supporting the dressing causing it to adopt the desired conformation. The dressing is then contacted to the body surface. When the dressing has been applied, the cover layer is easily removed and discarded.

To facilitate the release of the cover layer from the backing film next to the incision lines it is especially advantageous if the incision lines are curved or angular, as this will provide a flap or grip when the dressing is bended.

Preferably one of the incisions lines are longer than the other as this will create a pocket or strap having a conical conformation, which will make the dressing able to adopt a very concave conformation which is especially advantageous when treating sacral wounds.

The invention also relates to a method of application of a dressing comprising a proximal wound contacting surface covered wholly or partly by a skin-friendly adhesive, a distal surface comprising a non-adhesive backing film, said backing film at least partly being covered by a cover layer wherein said cover layer comprises at least one incision line, and wherein said incision line stretches over the surface of the dressing leaving an uninterrupted zone at the edge of the dressing wherein the dressing is bended towards the cover layer, so the part of the cover layer next to the incision line will rise to form a pocket or a strap in which a body part is inserted so as to support the dressing and cause it to adopt a desired conformation, and the dressing is then brought into contact with the body surface.

When bending the dressing, a pocket or strap is often formed automatically. If desired, the cover layer next to the incision line may be released from the backing film before bending.

The invention furthermore relates to a method of preparing a dressing comprising a proximal wound contacting surface covered wholly or partly by a skin-friendly adhesive, a distal surface comprising a non-adhesive backing film, said backing film at least partly being covered by a cover layer wherein said cover layer comprises at least one incision line, and wherein said incision line stretches over the surface of the dressing leaving an uninterrupted zone at the edge of the dressing wherein individual dressings are cut from a stock laminate, characterised in that at least one incision line is produced in the cover layer in line with the final confectioning and cutting of individual dressings.

The wound dressing according to the invention is typically comprising of a elastomer film which is coated on one side with an adhesive. To protect the adhesive is a siliconised release liner covering this side of the dressing, optionally with a folded non-touch siliconised release liner covering a part of the product. On top of the elastomer film is typically a cover layer, which may serve as a carrier layer during the manufacturing process. The cover layer may be left on the dressing as a protection layer to be removed by the user before applying the dressing, or it may be removed at the end of the manufacturing process.

The distance between the incision lines is of importance for the ability of the dressing to adopt a desired conformation, as well as the length of the lines is important, one is preferably longer than the second. Thus, a pocket of a conical conformation is readily formed, which is highly desirable as this conformation offers the best grip and ability to impart a desired conformation to the dressing. With a short distance between the incision lines, the strap will be thin and can be difficult to control, while when the distance between the incision lines is larger, the strap can become stiff and decrease the flexibility of the dressing. The optimal distance will highly depend on the material chosen for the cover layer. The optimal distance for a siliconised paper will be from 1-5 cm, more preferred from 2-4 cm.

The skin-friendly adhesive may cover all of the proximal wound contacting surface or only a part thereof and may be any skin-friendly adhesive known per se, e.g. an adhesive comprising hydrocolloids or other moisture absorbing constituents for prolonging the time of use. The adhesive may suitably be of the type disclosed in GB patent specification No. 1 280 631, in DK patent specifications Nos. 127,578, 148,408, 154,806, 147,226 and 154,747, in EP published application Nos. 0 097 846 and 0 415 183, in SE published application No. 365,410, in WO publication No. 88/06894, in US patent specification No. 4,867,748, and in NO published application No. 157,686. Especially preferred are the adhesives disclosed in US patent Nos. 4,367,732 and 5,051,259 and DK patent specification No. 169,711.

In one embodiment of the invention, the proximal wound contacting surface of the dressing is devoid of adhesive.

The backing film may be of any suitable material known per se for use in the preparation of wound dressings e.g. a foam, a nonwoven or a polyurethane, polyethylene, polyester or polyamide film. The backing film may be water impervious or permeable for water or it may be of a type having a higher water permeability when in contact with liquid water than when not in contact.

A suitable material for use as a film is a polyurethane. A preferred low friction film material is disclosed in US patent No. 5,643,187.

The cover film may optionally be paper, optionally treated with silicone, wax or Teflon, polyethylene terephthalate, high density polyethylene, medium density polyethylene, low density polyethylene, polypropylene, nylon or the like.

A dressing according to the invention may be prepared by a manner known per se for the preparation of medical dressings by substituting the raw materials and it will be routine for the skilled in the art to adapt the process parameters to the actual materials.

The dressing of the invention preferably has bevelled edges in order to reduce the risk of "rolling-up" the edge of the dressing. A bevelling may be carried out discontinuously or continuously in a manner known per se e.g. as disclosed in EP patent No. 0 264 299 or in US patent No. 5,133,821.

Furthermore, the dressing of the invention may comprise a non-touch grip known per se for applying the dressing to the skin without touching the adhesive layer. Such a non-touch grip is not present after application of the dressing.

### DETAILED DESCRIPTION OF THE DRAWINGS

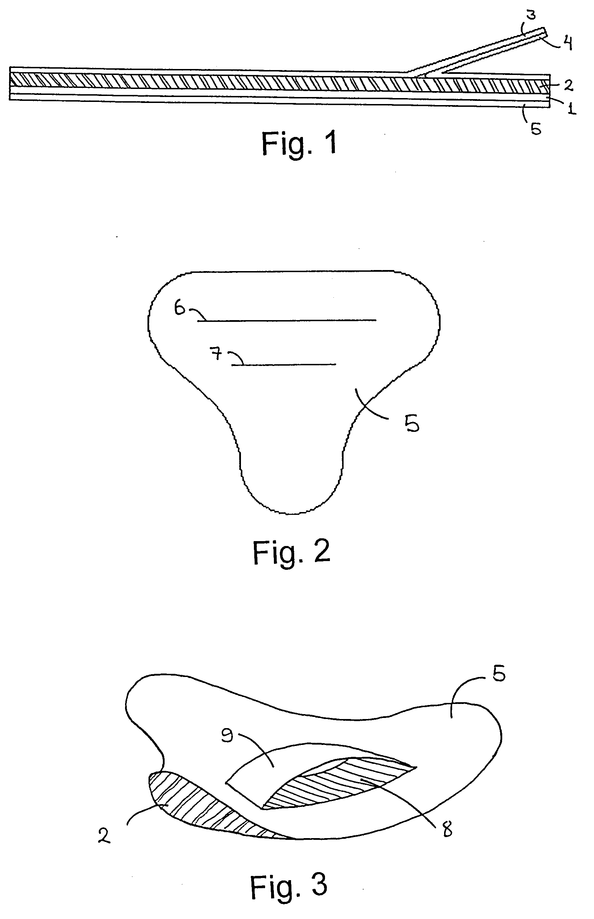

A preferred embodiment of the invention is shown in a cross-section in figure 1. A dressing comprises a non-adhesive film (1) forming a backing film coated with adhesive (2) on the proximal side. A siliconised release liner (3) is covering the adhesive part, and a folded siliconised non-touch release liner (4) is covering a part of the product. On the distal side of the film (1) is a cover layer (5), such as siliconised paper, covering at least a part of the surface of the film (1).

Figure 2 is showing the same dressing seen from the distal side, the dressing has a general triangular shape as preferred in treatment of sacral wounds. In the cover layer (5) are two incision lines (6,7) which incision lines stretches over the surface of the dressing leaving an uninterrupted zone at the edge of the dressing. The first line (6) is preferably longer than the second line (7).

In figure 3 is shown the same dressing being prepared for application, the part of the cover layer between the incision lines (6,7) has, if necessary, been released and the dressing is bended towards the cover layer (5), which causes the released part or strap of the layer (9) to rise and form a pocket (8) or strap into which a body part, such as a hand or fingers, can be inserted.

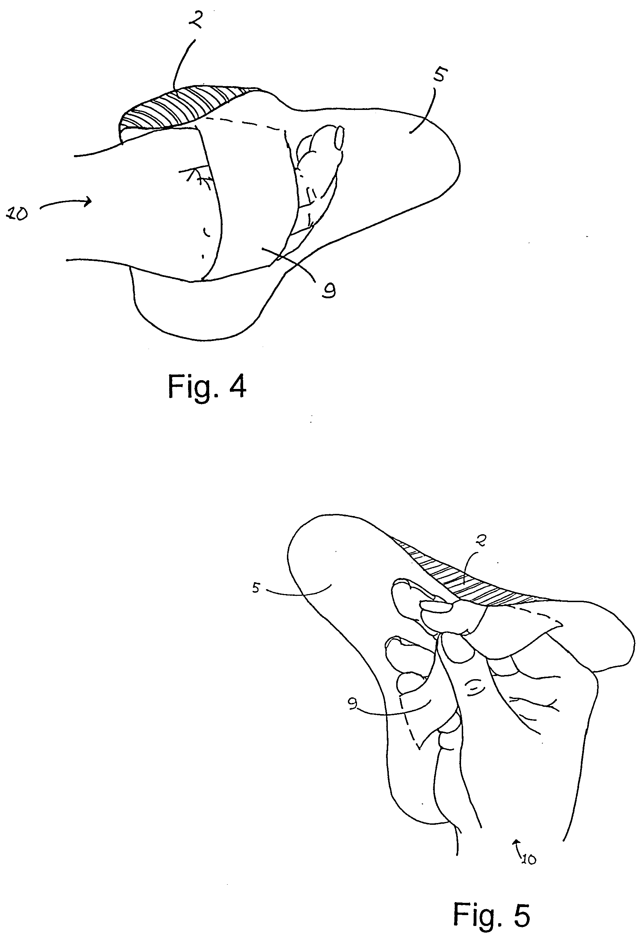

Figure 4 is showing the dressing when the release liner has been removed from the adhesive part and the hand (10) or a part thereof is inserted in the pocket (8) or strap formed by the cover layer between the incision lines to support the dressing and causing it to adopt the desired conformation. The hand can be inserted in the pocket with the palm of the hand facing the dressing as shown in figure 4 and the curved conformation of the dressing can be controlled by curving the hand or the fingers.

Another way of handling the dressing is to insert the hand in the pocket or strap with the palm of the hand facing away from the dressing as shown in figure 5. By inserting two or more fingers in the pocket and pressing the thumb against the released strap (9) of the cover layer, a better control of the dressing's conformation is obtained, the dressing can be formed into a extremely concave conformation which is especially desired before placing a dressing when in the treatment of sacral wounds as it may be difficult otherwise to ensure full contact with the wound and the surrounding area of skin in this area normally being retracted as compared with the surrounding areas. In this position the dressing is easily handled with one hand.

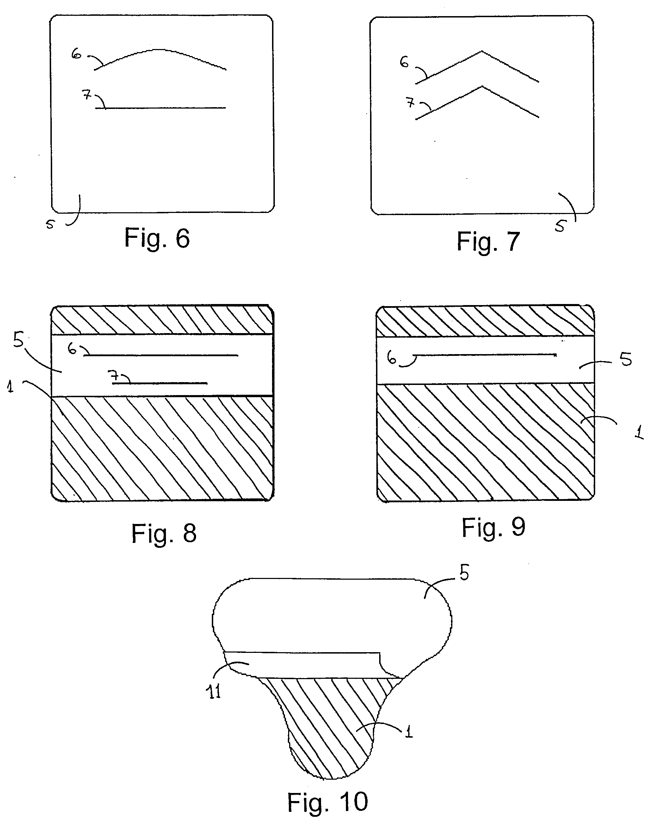

Figure 6 shows another embodiment of the invention, where one of the incision lines (6) are curved.

Figure 7 shows a third embodiment of the invention, where the incision lines (6,7) are rectilinear.

Figure 8 shows fourth embodiment of the invention, where the cover layer is divided so that a part thereof may be removed before applying the dressing. The part of the cover layer (5) having the incision lines (6,7) are used for application.

Figure 9 shows fifth embodiment of the invention, where the dressing is partly covered by the cover layer (5) and only comprises one incision line (6).

The embodiments of the invention shown i figure 8 and figure 9 are especially advantageous, when using a dressing which is not coated with adhesive. In this case the dressing is brought in contact with the wound, and may be secured to the body with adhesive tape or film across the non-covered part of the dressing. Then the cover layer can be removed.

Figure 10 shows sixth embodiment of the invention, where the dressing is covered by the cover layer, wherein the cover layer has been released along the cut-off edge and folded up to form a handle (11).

## Claims

1. A dressing comprising a proximal wound contacting surface covered wholly or partly by a skin-friendly adhesive, a distal surface comprising a non-adhesive backing film, said backing film at least partly being covered by a cover layer wherein said cover layer comprises at least one incision line, and wherein said incision line stretches over the surface of the dressing leaving an uninterrupted zone at the edge of the dressing.

2. A dressing according to claim 1, characterised in that two incision lines are present in the cover layer.

3. A dressing according to any of claims 1-2 characterised in that said incision lines are linear or curved.

4. A dressing according to any of claims 1-3 characterised in that the incision lines are substantially parallel.

5. A dressing according to any of claims 1-4 characterised in that one of the incision lines are longer than the other.

6. A method of application of a dressing comprising a proximal wound contacting surface covered wholly or partly by a skin-friendly adhesive, a distal surface comprising a non-adhesive backing film, said backing film at least partly being covered by a cover layer wherein said cover layer comprises at least one incision line, and wherein said incision line stretches over the surface of the dressing leaving an uninterrupted zone at the edge of the dressing wherein the dressing is bended towards the cover layer, so the cover layer next to the incision line will rise to form a pocket or a strap in which a body part can be inserted so as to support the dressing and cause it to adopt a desired conformation, and the dressing is then brought into contact with the body surface.

7. A method of preparing a dressing comprising a proximal wound contacting surface covered wholly or partly by a skin-friendly adhesive, a distal surface comprising a non-adhesive backing film, said backing film at least partly being covered by a cover layer wherein said cover layer comprises at least one incision line, and wherein said incision line stretches over the surface of the dressing leaving an uninterrupted zone at the edge of the dressing wherein individual dressings are cut from a stock laminate, characterised in that at least one incision line is produced in the cover layer in line with the final confectioning and cutting of individual dressings.
